# EUROPÄISCHE PATENTSCHRIFT

(11) **EP 2 192 911 B1**
(45) Veröffentlichungstag und Bekanntmachung des Hinweises auf die Patenterteilung: **19.09.2018**
(21) Anmeldenummer: 08833341.4
(22) Anmeldetag: 26.09.2008
(51) Int. Cl.: A61K 36/185, A61P 17/00

(54) **PFLANZENEXTRAKT AUS THC-ARMEN CANNABIS ZUR BEHANDLUNG VON ERKRANKUNGEN**
PLANT EXTRACT FROM LOW-THC CANNABIS FOR THE TREATMENT OF DISEASE
EXTRAIT DE PLANTES DE CANNABIS À FAIBLE THC POUR LE TRAITEMENT DE MALADIES

(30) Priorität: 26.09.2007 DE 102007046086
(43) Veröffentlichungstag der Anmeldung: 09.06.2010
(73) Patentinhaber: Bionorica SE, 92318 Neumarkt (DE)
(72) Erfinder: KLÄGER, Regina, 86889 Landsberg (DE); KLÄGER, Rudolph, 86889 Landsberg (DE)
(74) Vertreter: Simandi, Claus
(86) Internationale Anmeldenummer: PCT/DE2008/001581
(87) Internationale Veröffentlichungsnummer: WO 2009/039843

(56) Entgegenhaltungen:
- WO-A-02/32420
- WO-A-02/069993
- WO-A-2005/072719
- DE-C1- 19 833 567
- US-B1- 6 403 126
- VOGL C.R. ET AL.: "Hemp (Cannabis sativa L.) as a Resource for Green Cosmetics: Yield of Seed and Fatty Acid Compositions of 20 Varieties Under the Growing Conditions of Organic Farming in Austria" JOURNAL OF INDUSTRIAL HEMP, Bd. 9, Nr. 1, 2004, Seiten 51-68, XP002536675
- FIOCCHI A ET AL: "The efficacy and safety of gamma-linolenic acid in the treatment of infantile atopic dermatitis" JOURNAL OF INTERNATIONAL MEDICAL RESEARCH, Bd. 22, Nr. 1, 1994, Seiten 24-32, XP008108315 ISSN: 0300-0605

## Beschreibung

Die vorliegende Erfindung betrifft einen Pflanzenextrakt aus einer Tetrahydrocannabinol-(THC-) armen Sorte von *Cannabis-sativa subsp. sativa* zur Behandlung von Erkrankungen. Die Erfindung betrifft weiterhin die Herstellung des Extrakts sowie pharmazeutische oder topische Zusammensetzungen, die diesen Extrakt enthalten und deren Verwendungen.

Die Pflanzengattung *Cannabis* gehört zu der Familie der Hanfgewächse und besteht nach neueren Erkenntnissen aus einer einzigen Art *(Cannabis sativa),* die in drei Unterarten vorkommt: Nutzhanf *(Cannabis sativa* subsp. *sativa)* L., Indischer Hanf *(Cannabis sativa* subsp. *indica)* (Lam.) und Ruderalhanf *(Cannabis sativa* var. *spontanea*)*.* Je nach Verwendungszweck wird zwischen Rausch-bzw. Medizinalhanf mit dem Wirkstoff THC sowie Nutz-und Schmuckhanf unterschieden.

Cannabis ist als nachwachsender Rohstoff wegen seiner problemlosen Zucht und vollständigen Nutzbarkeit beliebt. Es werden keinerlei Herbizide benötigt, da die Pflanzen bereits nach wenigen Tagen den Boden vollständig beschatten, so dass kein Unkraut mehr Licht findet. Außerdem ist Cannabis äußerst schädlingsresistent und pflegeleicht. Cannabis produziert mehr Biomasse als jede andere heimische Nutzpflanze, ist in der Wirtschaft äußerst vielseitig einsetzbar und wird wegen seiner hohen Haltbarkeit, Umweltverträglichkeit und niedrigen Energiebilanz geschätzt.

Aus Cannabis können eine Vielzahl verschiedener Produkte aus allen Bereichen des täglichen Lebens hergestellt werden, wie z.B. Werkstoffe (Baustoffplatten, Dämmstoffe, Dichtmaterial etc), Kosmetika (Cremes, Massageöl, Seife etc.), Nahrungsmittel (Futtermittel, Öl, Margarine, Fette etc.), Öle, Ölprodukte (Öl zur Herstellung von Druck-bzw. Ölfarben sowie Kitt-und Spachtelmassen, Kunststoffe aus Öl, Tenside), Papiere, Vliese, Zellstoffe, natürliche Dämmstoffe und Stoffe (Kurz-und Langfasern).

In den Cannabispflanzen findet man als Inhaltsstoffe die sogenannten Cannabinoide. Man schätzt, dass das Harz der Cannabispflanze über 70 verschiedene Cannabinoide enthält, wovon einige, z.B. THC, psychotrope Wirkungen haben. Die Zielstrukturen der Cannabinoide bei Aufnahme in den menschlichen Organismus sind die Cannabinoid-Rezeptoren CB-1 und CB- 2 des Endocannabinoid-Systems. Physiologischer Ligand dieser Rezeptoren ist das Arachidonsäure-Derivat Anandamid.
Derzeit ist vor allem das Cannabinoid THC als medizinischer Wirkstoff bekannt. So ist z.B. das halbsynthetische THC, Dronabinol, in Deutschland und anderen Staaten als verschreibungspflichtiges Betäubungsmittel (Handelsname *Marinol* ® bei Anorexie und Kachexie bei HIV-und AIDS-Patienten, sowie als Antiemetikum bei Übelkeit und Erbrechen unter Zytostatika-bzw. Bestrahlungstherapie im Rahmen einer Krebstherapie anwendbar. Das vollsynthetische THC-Derivat Nabilon hat eine ähnliche Indikation. Außerdem befindet sich THC in der klinischen Erprobungsphase für die Behandlung von Glaukomen und Autoimmunerkrankungen, wie Multipler Sklerose, Morbus Crohn oder Colitis ulcerosa.
Ein weiteres bekanntes, allerdings nicht psychoaktives Cannabinoid aus weiblichen Hanfpflanzen von *Cannabis sativa* ist Cannabidiol (CBD). Medizinisch wirkt es krampf-und angstlösend, entzündungshemmend, gegen Übelkeit sowie Augeninnendruck-senkend.

WO 2005/072719 beschreibt einen Pflanzenextrakt, der mindestens ein saures Cannabinoid enthält und zugleich einen geringen THC-Gehalt von 0-5 Gew.-% aufweist. Dieser Pflanzenextrakt wird vorzugsweise aus den Blüten der Sorten *Cannabis sativa* subsp. *sativa* und *Cannabis sativa* subsp. *indica* mittels Flüssigextraktionsverfahren, vorzugsweise unter nicht-decarboxylierenden Bedingungen, gewonnen. Die nicht-decarboxylierenden Bedingungen sind notwendig, um den THC-Gehalt möglichst gering zu halten. Daneben wird zwar die Verwendung der Pflanzenextrakte für die Herstellung eines Medikaments zur Behandlung von entzündlichen Hauterkrankungen, wie z.B. Dermatitis oder Psioriasis erwähnt, allerdings ohne jegliche experimentellen Daten, die die Wirksamkeit belegen könnten.

WO02/32420 offenbart ein Verfahren zur Herstellung eines Cannabisextrakts aus THC-armen Cannabis Sorten umfassend die Schritte des Trocknens, Zerkleinerns und Extrahierens, sowie entsprechende Extrakte zur Herstellung eines Medikaments zur Behandlung von Krankheiten wie Asthma.

Darüber hinaus wird in US 2007/0060639 eine pharmazeutische Zusammensetzung offenbart, die mindestens ein trizyklischen Cannabinoid für die intranasale Verabreichung umfasst. Des Weiteren wird die Verwendung dieser pharmazeutischen Zusammensetzung bei allergischer Rhinitis beschrieben.

Ein Beispiel eines Verfahrens zur Herstellung eines Cannabisextraktes wird in der US 2003/0017216A1 offenbart. Hierfür wird das Pflanzenmaterial mit einem Lösungsmittel, z.B. Isopropanol, für eine bestimmte Zeitdauer versetzt. Dabei ist die Zeitdauer geringer als die, die nötig wäre, um eine Gleichgewichtskonzentration an gelösten Cannabinoiden im Lösungsmittel zu erhalten. Anschließend werden das Lösungsmittel und die gelösten Cannabinoide von dem Pflanzenmaterial abgetrennt.

Bisher völlig vernachlässigt und nicht untersucht ist die medizinische Wirksamkeit eines Pflanzenextrakts aus der THC-armen Sorte *Cannabis sativa subsp. sativa.* Ein derartiger Pflanzenextrakt hätte nicht nur den Vorteil der kostengünstigen Bereitstellung; durch den legalen Anbau von THC-armen Sorten, wie z.B. Futura 75, ferner wird vor allem ein eventueller Konflikt hinsichtlich des Betäubungsmittelgesetzes gänzlich umgangen.

Erkrankungen des allergischen und immunologisch bedingten Formenkreises werden derzeit überwiegend mit den nebenwirkungslastigen Cortisonpräparaten (auch als Glucocorticoidpräparate bekannt) oder Calcineurin-Inhibitoren (aus der Klasse der Cytostatika) behandelt. Allerdings wird die topisch behandelbare Ausdehnung der Hautfläche sowohl bei den Cortisonpräparaten als auch bei den Calcineurin-Inhibitoren dadurch begrenzt, dass bei großflächiger Applikation mit systemisch relevanter Resorption über das behandelte Hautareal gerechnet werden muss. Damit kommen die Nebenwirkungen beider Therapieprinzipien im gesamten Körper klinisch relevant zum Tragen.

Neben der Einschränkung der Ausdehnung der behandelbaren Hautfläche ist auch die Therapiedauer begrenzt. Allerdings handelt es sich bei vielen Cortison-pflichtigen bzw. nur durch Calcineurin-Inhibitoren behandelbare Krankheitserscheinungen, wie z. B. bei Neurodermitis, um chronische und damit dauerhaft behandlungsbedürftige Erkrankungen mit einem erheblichen Leidensdruck der betroffenen Patienten. Die Behandlung mit Calcineurin-Inhibitoren ist auf maximal vier Wochen pro Zyklus begrenzt. Ähnliches gilt für die Behandlung mit Cortison-Präparaten. In beiden Fällen muss nach dem zur Vermeidung schwerwiegender und teilweise irreversibler Nebenwirkungen medizinisch erforderlichen Absetzen der Therapie mit einer akuten und für den Patienten überaus belastenden Exacerbation seiner Symptome ("Reboundeffekt") gerechnet werden.

Für die Behandlung mit Cortison gibt es -bezogen auf die lokalen Nebenwirkungen -kein starres Schema. Doch muss grundsätzlich mit einer erheblichen Atrophie der behandelten Hautareale gerechnet werden. Neben kosmetisch, die Lebensqualität einschränkenden Hautveränderungen ("Lederhaut"), die vor allem bei Krankheitsbefall im Gesichtsbereich erhebliche Relevanz haben, werden auch funktionell relevante Hautveränderungen beobachtet. Die Hautatrophien gehen vor allem mit einer krankhaft gesteigerten Verletzlichkeit der papierdünnen Hautareale einher.

Obwohl sowohl die Cortisonpräparate als auch die Calcineurin-Inhibitoren bei dem überwiegenden Anteil an Patienten eine gute Wirksamkeit zeigen, ist die Compliance aufgrund der oben erwähnten und damit befürchteten Nebenwirkungen relativ gering. Häufig nehmen die Patienten die Symptome ihrer Erkrankung aus Angst vor den Nebenwirkungen der genannten Substanzgruppen in Kauf.

Es ist somit Aufgabe der vorliegenden Erfindung, ein wirksames und nebenwirkungsfreies Medikament zur Behandlung von Erkrankungen, vorzugsweise zur Behandlung von Erkrankungen des allergischen und immunologisch bedingten Formenkreises, auf pflanzlicher Basis bereitzustellen, das nicht nur kostengünstig und einfach in seiner Bereitstellung ist, sondern das auch aufgrund seines schnellen und überzeugenden Wirkungseintritts und (bislang) keinen Nebenwirkungen eine sehr hohe Compliance bei den Patienten erzielt. In einem ersten Aspekt wird die Aufgabe der vorliegenden Erfindung durch einen Pflanzenextrakt aus den Blüten und blütennahen Blättern und/oder Stengeln und/oder Wurzeln und/oder Samen, bevorzugt aus den Blüten und blütennahen Blättern, einer Tetrahydrocannabinol-(THC-) armen Sorte von *Cannabis sativa subsp. sativa* zur Behandlung von Erkrankungen gelöst (nachstehend "Pflanzenextrakt").

Im Rahmen dieser Erfindung wird unter einer "Tetrahydrocannabinol-(THC-) armen Sorte" eine solche verstanden, die in einem solchen Pflanzenextrakt weniger als 5 %, vorzugsweise weniger als 2 %, insbesondere weniger als 0,5 bis 0,2 % oder bis 0,1 oder 0 % (Gew. %) THC - Gehalt aufweist. Insbesondere sind solche Mengen THC darunter zu verstehen, die keine klinisch relevante Konzentration darstellt.

Überraschenderweise konnte durch die im Rahmen der Erfindung durchgeführten Versuche gezeigt werden, dass dieser Pflanzenextrakt eine mit Cortison und Calcineurin vergleichbare oder sogar überlegene Wirksamkeit (ohne deren Nebenwirkungen!) hinsichtlich Erkrankungen des allergischen und immunologisch bedingten Formenkreises zeigt. Bei Patienten mit klinisch hochdramatisch verlaufender Neurodermitis konnte das im Vordergrund fast aller Patienten stehenden Bedürfnis nach Juckreizstillung im Vergleich zu den vorher verordneten Cortison-bzw. Calcineurin-Inhibitoren geradezu dramatisch verbessert werden. Die klinische Relevanz ergibt sich aus den Sekundärfolgen dieses für die Neurodermitis kardinalen Leitsymptoms: blutig kratzen an den betroffenen Hautarealen wegen des unstillbaren Juckreizes mit konsekutiver Lichenifikation. Im Gegensatz zu der nur innerhalb von Tagen einsetzenden Symptom Beeinflussung (Juckreiz) nach Cortison-Therapie kann mit dem THC-armen Pflanzenextrakt in einer pharmazeutisch geeigneten Darreichungsform das Leitsymptom Juckreiz innerhalb von wenigen Minuten gebessert werden. Durch diese Verbesserung der im Vordergrund stehenden klinischen Symptomatik "Juckreiz" werden auch Sekundär-Symptome wie kratzbedingte Hautläsionen mit Blutungen, gefolgt von anschließender Lichenifikation, innerhalb weniger Tage klinisch sichtbar gebessert. Dies ist vor allem bei Frauen bzw. im Gesichtsbereich von höchster Bedeutung.

Darüber hinaus zeigt dieser Pflanzenextrakt bislang keinerlei Nebenwirkungen. Somit sind all die unter der Behandlung mit Cortison oder Calcineurin-Inhibitoren oben erwähnten Nebenwirkungen nicht zu befürchten bzw. können diese insbesondere bezogen auf die topischen Nebenwirkungen einer längeren Cortisontherapie sogar geheilt werden. Die Verwendung von Cortison und Calcineurin-Inhibitoren kann komplett vermieden werden. Damit stellt sich für Patienten, die bisher für die genannten Substanzklassen entweder therapieresistent oder bei denen Nebenwirkungen bereits gravierend waren oder welche die Therapie mit diesen Substanzgruppen abgelehnt haben, ein völlig neuer Therapieansatz dar.

Des Weiteren konnte eine klinisch eindeutige, aber bisher auch nicht vermutete antimykotische Wirkung gezeigt werden, die sich sowohl topisch als auch systemisch als sehr wertvoll -vor allem wegen ihres raschen Wirkungseintrittes -im weiteren Therapiespektrum erweisen dürfte. Diese Wirksamkeit bezieht sich sowohl auf Saprophyten, als auch auf Dermatophyten und Hefepilze. Die Relevanz dieser antimykotischen Wirkung ergibt sich, über die bisher nebenwirkungsarme bis -freie Wirksamkeit hinaus, sowohl aus der Notwendigkeit der Anwendung von Antimykotika wie Metronidazol (sowohl topisch wie systemisch) bei verschiedenen Hauterkrankungen als auch deren nicht unerhebliches Nebenwirkungspotential.

Gemäß der oben aufgezeigten überraschenden Wirksamkeit des Pflanzenextrakts der vorliegenden Erfindung wird dieser Extrakt für die Herstellung eines Arzneimittels bevorzugt zur Behandlung von Neurodermitis, Kontaktekzem, Allergien, der Prävention oder Behandlung von fototoxischen Reaktionen, der Behandlung von entzündlichen, juckenden Dermatosen, Rosazea, perioraler Dermatitis, Akne, Akne conglobata, Psoriasis, (vulgaris, arthropathica, pustulosa), Mückenstichen, Hautatrophien (insbesondere auch Cortison-bedingte Hautveränderungen), allergischer Rhinitis, Privinismus Konjunktivitis, Otitis externa, Asthma bronchiale, Morbus Crohn, Colitis ulcera, Sarkoidose, oder entzündlich-rheumatischen Erkrankungen der Weichteile oder Gelenke sowie Mykosen verwendet. Unter entzündlichen, juckenden Dermatosen im Sinne der vorliegenden Erfindung werden insbesondere Erkrankungen ausgewählt aus der Gruppe bestehend aus Rosazea, Periorale Dermatitis, Psoriasis vulgaris, Psoriasis pustulosa, Akne, Akne conglobata verstanden.

Unter entzündlich-rheumatischen Erkrankungen im Sinne der vorliegenden Erfindung werden Erkrankungen ausgewählt aus der Gruppe bestehend aus chronische Polyarthritis, Morbus Bechterew, Psoriasis arthritis, Polymyalgia rheumatica, Kollagenosen und Vaskulitiden, verstanden.

Im Rahmen dieser Erfindung wird unter dem Begriff "Neuroderm(at)itis" (auch besser: atopische Dermatitis bzw. "atopisches Ekzem") eine immer häufiger auftretende schubweise Hauterkrankung, die insbesondere Heranwachsende betrifft verstanden. Die Haut eines an Neurodermitis Erkrankten ist in der Akutphase hoch sensibel und kann allergisch auf psychische und physische Stressoren sowie eine ganze Reihe von individuell unterschiedlichen Umweltfaktoren und -noxen reagieren. In der Akutphase ist die Haut entzündet und der Betroffene leidet ganz besonders unter dem quälenden Juckreiz. Soweit bekannt, liegen der Neurodermitis keine Erkrankung der inneren Organe zugrunde, sondern entzündliche freie radikale produzierende Prozesse der äußeren Hautschichten, so dass durch externe Applikation in einer topischen Anwendungsform mittels einer dermatologischen oder kosmetischen Zusammensetzung z.B. in Form von Salben, Cremes oder Gelen hier Einfluss positiv genommen werden kann.

"Mykosen" im Sinne der vorliegenden Erfindung sind Pilzerkrankungen, die meistens durch Fadenpilze oder Sprosspilze ausgelöst werden. Man unterscheidet hierbei zwischen oberflächlichen und systemischen Mykosen. Oberflächliche Mykosen können den ganzen Körper befallen, am bekanntesten ist der Fußpilz (Tinea pedis), aber auch die Schleimhäute (Soor bzw. Kandidose) können betroffen sein.

Ferner können Mykosen und Neurodermitis nebeneinander vorliegen, insbesondere in Hautfaltungen (Kniekehle etc.).

Der Pflanzenextrakt der vorliegenden Erfindung kann durch jegliches, dem Fachmann bekanntes Extraktionsverfahren gewonnen werden. Für Auszüge, die zugleich als direkt anwendbare flüssige Arzneiformen verwendet werden können, eignen sich die folgenden Extraktionsmittel: kaltes Wasser, Kochsalzlösung, verdünnte Essigsäure, Südwein, Ethanol-Wasser-Gemische, Ethanol, niedermolekulare sonstige Alkohole, Aceton, Ester, Ether und Mischungen davon. Zur Gewinnung von Trockenextrakten sind Methanol, organische Lösungsmittel wie Aceton, Ether, Dichlormethan sowie überkritische Gase, Vakuumextraktion und Gefriertrocknung gebräuchlich und dem Fachmann bekannt. Es können hierbei zwischen einfachen Extraktionsmethoden ausgewählt aus der Gruppe bestehend aus ruhender Mazeration, Bewegungsmazeration, Digestion, Perkolation, Reperkolation, Evakolation und Diakolation und spezielle Extraktionsverfahren ausgewählt aus der Gruppe bestehend aus Kombination von Mazeration und Perkolation, Ultraschallextraktion, Gegenstromextraktion und Extraktion mit Separatoren, Zentrifugen und Dekantern gewählt werden. Diese Verfahren sind dem Fachmann bekannt und es wird beispielhaft auf Hagers Handbuch der Pharmazeutischen Praxis (5. Auflage, Bd. 2; S 1026-1030 Springer Verlag, Berlin-Heidelberg-New York (1991)) verwiesen. Als Ausgangsmaterial können frische Pflanzen oder Pflanzenteile eingesetzt werden, üblicherweise wird jedoch von getrockneten Pflanzen und/oderPflanzenteilen ausgegangen, die vor der Extraktion mechanisch zerkleinert werden können. Hierbei eignen sich alle dem Fachmann bekannten Zerkleinerungsmethoden, als Beispiel sei das Zerstoßen mit einem Mörser genannt.

Als Lösungsmittel für die Durchführung der Extraktionen können, wie oben erwähnt, alle Lösungsmittel mit einer gewissen Polarität, vorzugsweise organische Lösungsmittel, Wasser (destilliert oder nicht destilliert) oder Gemische aus organischen Lösungsmitteln und Wasser, insbesondere niedermolekulare Alkohole, Ester, Kohlenwasserstoffe, Ketone oder halogenhaltige Kohlenwasserstoffe mit mehr oder weniger hohen Wassergehalten verwendet werden. Beispiele sind protische (Wasser, Alkohole, Säuren, primäre und sekundäre Amine) und aprotische (Acetonitril, Dimethylformamid, Dimethylsulfoxid, Hexamethylphosphorsäuretriamid, Nitromethan, tert-Amine) Lösungsmittel. Besonders bevorzugt ist die Extraktion mit Wasser, Methanol, Ethanol, Propanol, Isopropanol, Pentan, Hexan, Heptan, Aceton, Chloroform, Propylenglycolen, Polyethylenglycolen, Ethylacetat, Dichlormethan, Trichlormethan sowie Mischungen davon. Die Extraktion erfolgt in der Regel bei 15 bis 25°C (wässrige Extrakte) oder bei 20 bis 35°C (niedermolekulare Alkohole), im Prinzip bevorzugt bei Raumtemperatur, um temperatursensible Extraktbestandteile zuverlässig zu schützen. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe unterzogen werden. Auch können die Extrakte anschließend beispielsweise einer Sprüh-oder Gefriertrocknung unterworfen werden. Unter dem Begriff "Extrakt" gemäß der vorliegenden Erfindung wird demnach ein Stoff und/oder Stoffgemisch verstanden, der/das durch einen oder mehreren Extraktions-und/oder anderen Verfahrensschritten aus der Hanfpflanze gewonnen wird.

In einer besonders bevorzugten Ausführungsform wird der Pflanzenextrakt der vorliegenden Erfindung mit einem Extraktionsmedium, Lösungsmittel und/oder Gemisch von Lösungsmitteln gewonnen, ausgewählt aus der Gruppe bestehend aus Wasser, Kochsalzlösung, niedermolekularen Alkoholen, Aceton, Estern und Ethern. Dabei wird vorzugsweise als Extraktionsmedium 0.9 % Kochsalzlösung, Ethanol oder Isopropanol verwendet, bevorzugt 90% Äthanol oder 70% Isopropanol.

Nach einer bevorzugten Ausführungsform der vorliegenden Erfindung wird für die Gewinnung des Pflanzenextrakts die THC -arme Cannabis sativa subsp. sativa Sorte Futura 75 verwendet. Ein weiterer Aspekt der vorliegenden Erfindung betrifft ein Verfahren zur Herstellung eines Pflanzenextraktes aus den Blüten und blütennaher Blätter einer THC-armen Cannabis sativa subsp. sativa, umfassend die Schritte von a) Trocknen der Blüten und/oder blütennahen Blättern und/oder Stengeln und/oder Wurzeln und/oder Samen, b) Zerkleinern der Blüten und/oderblütennahen Blättern und/oder Stengeln und/oderWurzeln und oder Samen und c) Extraktion der Blüten und/oder blütennahen Blättern und/oder Stengeln und/oder Wurzeln und/oder Samen. Bevorzugt wird das Verfahren mit Blüten und/oder blütennahen Blättern durchgeführt, da hier am meisten Wirkstoff enthalten ist.

Nach einer bevorzugten Ausführungsart umfasst die Extraktion die Schritte C-1) Versetzen der Blüten und blütennahen Blätter für 24h -48h oder 12h -48h mit einem Extraktionsmedium bei Raumtemperatur, und C-2) Filtrieren des Extraktes.

In einer noch weiter bevorzugten Ausführungsform umfasst das Verfahren der vorliegenden Erfindung den Schritt d) Gefriertrocknen des gewonnenen Extraktes und/oder VakuumExtraktion des gewonnenen Extraktes.
In einer bevorzugten Ausführungsform verwendet das Verfahren der vorliegenden Erfindung als Extraktionsmedium Lösungsmittel und/oder Gemische von Lösungsmitteln, ausgewählt aus der Gruppe bestehend aus Wasser, Kochsalzlösung, niedermolekularen Alkoholen, Aceton, Estern und Ethern, vorzugsweise 0.9 % Kochsalzlösung, Ethanol oder Isopropanol, bevorzugt 90% Äthanol oder 70% Isopropanol.

In einer besonders bevorzugten Ausführungsform wird in den Verfahren der vorliegenden Erfindung die THC-arme *Cannabis sativa subsp. sativa* Sorte Futura 75 verwendet. Sie kann aber auch durch jede andere *Cannabis sativa* Sorte mit einem nachdem jeweiligen Stand der nationalen gesetzlichen Regelungen (im deutschen Betäubungsmittelgesetz derzeit 0,2% THC-Gehalt) ersetzt werden.
Ein weiterer Gegenstand der vorliegenden Erfindung umfasst eine pharmazeutische Zusammensetzung, die einen Pflanzenextrakt, vorzugsweise aus den Blüten und blütennaher Blätter einer THC-armen *Cannabis sativa* Sorte, vorzugsweise die THC-arme *Cannabis sativa subsp. sativa* Sorte Futura 75, umfasst.

Ebenfalls betrifft die Erfindung eine kosmetische oder dermatologische Zusammensetzung in einer topischen Anwendungsform, die einen Pflanzenextrakt, vorzugsweise aus den Blüten und blütennaher Blätter einer THC-armen *Cannabis sativa* Sorte, vorzugsweise die THC-arme *Cannabis sativa subsp. sativa* Sorte Futura 75, umfasst.

In einer besonderen Ausführungsform umfasst die pharmazeutische oder dermatologische Zusammensetzung weiterhin pharmazeutisch geeignete Hilfs-und Zusatzstoffe. Die pharmazeutischen Mittel bzw. Zusammensetzungen der Erfindung werden mit üblichen festen oder flüssigen Trägerstoffen oder Verdünnungsmitteln und üblichen pharmazeutischen und technischen Hilfsstoffen entsprechend der gewünschten Applikationsart mit einer geeigneten Dosierung in an sich bekannter Weise hergestellt. Bevorzugte Zubereitungen bestehen in einer Darreichungsform, die zu einer topischen, oralen, inhalativen, intranasalen, enteralen oder parenteralen, beispielsweise i.p. (intraperitonealen), i.v. (intravenösen), i.m. (intramuskulären) oder perkutanen, Applikation geeignet ist. Solche Darreichungsformen sind beispielsweise Tabletten, Filmtabletten, Dragees, Pillen, Kapseln, Pulver, Cremes, Salben, Lotionen, Flüssigkeiten, wie Sirupe, Gele, injizierbare Flüssigkeiten, beispielsweise zur i.p., i.v., i.m. oder perkutanen Injektion, Nasensprays aber auch Inhalationssprays usw. Weiterhin sind auch Depotformen, wie implantierbare Zubereitungen, sowie Suppositorien geeignet. Dabei geben die einzelnen Zubereitungen die erfindungsgemäßen Extrakte je nach deren Art allmählich oder die gesamte Menge in kurzer Zeit an den Körper ab. Zur oralen Verabreichung können Kapseln, Pillen, Tabletten, Dragees und Flüssigkeiten oder andere bekannte orale Darreichungsformen als pharmazeutische Präparate eingesetzt werden. In diesem Falle können die Arzneimittel in der Weise formuliert sein, dass sie die Wirkstoffe entweder in kurzer Zeit freisetzen und an den Körper abgeben oder eine Depotwirkung aufweisen, so dass eine länger anhaltende, langsame Zufuhr von Wirkstoff zum Körper erreicht wird. Die Dosierungseinheiten können neben mindestens einem erfindungsgemäßen Pflanzenextrakt einen oder mehrere pharmazeutisch verträgliche Träger enthalten, beispielsweise Stoffe zur Einstellung der Rheologie des Arzneimittels, oberflächenaktive Stoffe, Lösungsvermittler,
Mikrokapseln, Mikropartikel, Granulate, Verdünner, Bindemittel, wie Stärke, Zucker, Sorbit und Gelatine, ferner Füllstoffe wie Kieselsäure und Talkum, Gleitmittel, Farbstoffe, Duftstoffe und andere Stoffe.

Entsprechende Tabletten können beispielsweise durch Mischen des erfindungsgemäßen Extraktes mit bekannten Hilfsstoffen beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Carboxypolymethylen, Carboxymethylcellulose, Celluloseacetatphthalat oder Polyvinylacetat erhalten werden. Die Tabletten können auch aus mehreren Schichten bestehen.

Entsprechend können Dragees durch Überziehen von analog zu Tabletten hergestellten Kernen mit üblicherweise in Drageeüberzügen verwendeten Mitteln, beispielsweise Polyvinylpyrrolidon oder Schellack, Gummi arabicum, Talk, Titanoxid oder Zucker, hergestellt werden. Dabei kann die Drageehülle aus mehreren Schichten bestehen, wobei die oben bei den Tabletten erwähnten Hilfsstoffe verwendet werden können.

Wirkstoffe können auch in Form einer Lösung formuliert werden, die für die orale oder topische Verabreichung bestimmt ist und die neben einem aktiven erfindungsgemäßen Pflanzenextrakt als Bestandteile ein pharmazeutisch verträgliches Öl und/oder eine pharmazeutisch verträgliche lipophile, oberflächenaktive Substanz und/oder eine pharmazeutisch verträgliche hydrophile, oberflächenaktive Substanz und/oder ein pharmazeutisch verträgliches wassermischbares Lösungsmittel enthält. Ebenso können Cremes, Salben, Lotionen und Tinkturen für die äußerliche Anwendung eingesetzt werden. In diesen Darreichungsformen sind häufig Hilfsstoffe enthalten, beispielsweise Stoffe zur Einstellung der Rheologie der Arzneimittel, oberflächenaktive Mittel, Konservierungsmittel, Lösungsvermittler, Verdünner, Stoffe zur Erhöhung der Permeationsfähigkeit für die erfindungsgemäßen Extrakte durch die Haut, Farbstoffe, Duftstoffe und Hautschutzmittel, wie Konditionierer und Feuchteregulatoren. Zusammen mit den erfindungsgemäßen Extrakten können auch andere Wirkstoffe in dem Arzneimittel enthalten sein (Ullmanns Enzyklopädie der technischen Chemie, Band 4 (1953), S. 1-39; J. Pharm. Sci. (1963) 52:918 ff., H. V. Czetsch-Lindenwald, Pharm. hd. (1961) 2:72 ff, Fiedler, Lexikon der Hilfsstoffe für Pharmazie, Kosmetik und angrenzende Gebiete, Cantor AG (1971)).
Entsprechende kosmetische Zusammensetzungen der erfindungsgemäßen Pflanzenextrakte können analog nach dem für den Fachmann üblich bekannten Formulierungen in einer topischen Anwendungsform bereitgestellt werden.

In einer weiteren Ausführungsform kann daher die erfindungsgemäße kosmetische oder dermatologische Zubereitung zur topischen Verwendung ("topische Zusammensetzung") in Form einer Salbe, Creme, Gel, Lotion (Hautmilch), Paste oder vorzugsweise Emulsion erfolgen. Ebenfalls sind wasserfreie Systeme möglich.
Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Sind die beiden Flüssigkeiten Wasser und Öl und liegen Öltröpfchen fein verteilt in Wasser vor, so handelt es sich um eine Öl-in-Wasser-Emulsion (O/W-Emulsion). Der Grundcharakter einer O/W-Emulsion ist durch das Wasser geprägt. Bei einer Wasser-in-Öl-Emulsion (W/O-Emulsion) handelt es sich um das umgekehrte Prinzip, in dem der Grundcharakter hier durch das Öl bestimmt wird. Weiterhin sind Mischsysteme wie Wasser-in-Öl-in-Wasser-Emulsionen (W/O/W-Emulsion) und Öl-in-Wasser-in-Öl-Emulsionen (O/W/O-Emulsionen) bekannt. Alle genannten Emulsionen sind erfindungsgemäß geeignet.
Zu den erfindungsgemäß geeigneten wasserfreien Systemen gehören reine Ölzubereitungen wie z.B. Hautöle. Ebenfalls einsetzbare Pasten enthaltend die erfindungsgemäße Zubereitung, zeichnen sich dadurch aus, das sie aus die gleichen oder ähnlichen Bestandteilen wie eine Emulsion besteht aber im wesentlichen wasserfrei sind. Im Rahmen der vorliegenden Erfindung werden die Begriffe Ölphase und Lipidphase synonym verwendet. In einer weiteren bevorzugten Ausführungsform kann die erfindungsgemäße Zubereitung als weiteren Bestandteil einen Emulgator beinhalten. In einer ganz bevorzugten Ausführung kann dieser Emulgator ein O/W-Emulgator sein.
Emulgatoren können vorteilhaft gewählt werden aus der Gruppe der nichtionischen, anionischen, kationischen oder amphoteren Emulgatoren.

Die erfindungsgemäßen Pflanzenextrakte können auch in geeigneten Lösungen, wie beispielsweise physiologischer Kochsalzlösung, als Infusions-oder Injektionslösung, Nasensprays, Augentropfen zur Anwendung kommen. Für eine parenterale Applikation können die Wirkstoffe in einem physiologisch verträglichen Verdünnungsmittel gelöst oder suspendiert sein. Als Verdünnungsmittel sind auch ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.
In einer besonderen Ausführungsform enthält die pharmazeutische oder dermatologische Zusammensetzung der vorliegenden Erfindung weitere Wirkstoffe bzw. Hilfsstoffe, vorzugsweise Dexpanthenol. Dexpanthenol fördert als Hilfsstoff bei wässrigen Zusammensetzungen, wie z.B. wässrigen Sprays, die Anhaftung. Bevorzugt wird Dexpanthenol bei Nasensprays, Mundsprays und Augentropfen/Augensalben eingesetzt. In einer weiteren Ausführungsform enthält die pharmazeutische Zusammensetzung der vorliegenden Erfindung zusätzlich eine pharmazeutisch effektive Menge des Proteins Filaggrin, entweder gemeinsam oder räumlich getrennt von dem erfmdungsgemäßen Pflanzenextrakt.

Filaggrin ist ein histidinreiches kationisches Protein, beziehungsweise eine Gruppe von isoformen Proteinen, die im Verhornungsprozess der Haut aus dem Profilaggrin hervorgehen. Filaggrin ist ein Protein der verhornenden Epithelzellen der Epidermis, das die Filamente aggregiert und das durch posttranslationale Modifikation des Profilaggrins in den Keratinozyten hervorgeht. Filaggrin besitzt strukturbildende Funktionen für die Epidermis. Ein auf einem genetischen beruhenden Filaggrinmangel wurde kürzlich als eine Ursache für die Entstehung einer Neurodermitis formuliert.

Ein weiterer Aspekt der vorliegenden Erfindung betrifft die Verwendung des Pflanzenextrakts oder der pharmazeutischen Zusammensetzung für die Herstellung eines Arzneimittels zur Behandlung von Neurodermitis, Allergien, entzündlichen, juckenden Dermatosen, Mückenstichen, Hautatrophien, allergischer Rhinitis, Privinismus, Otitis externa, Asthma bronchiale, Morbus Crohn, Colitis ulcera, Sarkoidose, Konjunktivitis, entzündlich-rheumatische Erkrankungen sowie Mykosen oder der Prävention oder Behandlung von phototoxischen Reaktionen. Vorzugweise wird der Pflanzenextrakt oder die pharmazeutische Zusammensetzung bei topischen Anwendungen zusammen mit einer pharmazeutisch effektiven Menge des Proteins Filaggrin verwendet.

In einem anderen Aspekt betrifft die vorliegende Erfindung die Verwendung des erfindungsgemäßen Pflanzenextrakts als Medikament zur effektiven Behandlung von Patienten, die auf eine Therapie mit einer herkömmlichen Cortisondosis nicht unzureichend oder schlecht, ansprechen bzw. Cortisonbehandlung ablehnen und/oder Nebenwirkungsbedingt nicht mit Cortison-haltigen Präparaten behandelt werden können. In diesen Fällen kann herkömmlich nur auf die (neben den Cortisonen) stärksten bisher bekannten Therapeutica -Calcineurin-Inhibitoren -ausgewichen werden, die sehr häufig nur zu einer unzureichenden Symptomlinderung der die Patienten subjektiv äußerst quälenden Symptome führen. Das die Behandlung außerdem noch mit schweren Nebenwirkungen behaftet sein kann, problematisiert die Situation noch weiter. Deshalb haben wir nach einer Möglichkeit gesucht, Calcineurin-Inhibitoren durch wirksamere und besser verträgliche Medikamente zur Behandlung der Neurodermitis ebenso wie auch umgekehrt Calcineurine zu ergänzen bzw. zu ersetzen. Die vorliegende Erfindung stellt somit eine wirksame und nebenwirkungsfreie Therapie dieser Patienten (Im Rahmen der vorliegenden Erfindung als "Therapieversager" bezeichnet) zur Verfügung. Diese Therapieversager können, wie oben angegeben, anstelle von Cortison effektiv mit dem erfindungsgemäßen Cannabisextrakt therapiert werden, so dass nicht auf die Calcineurin-Inhibitoren ausgewichen werden muss - und umgekehrt.
Daher betrifft die Erfindung ebenfalls eine Ersatztherapie zur Cortison- und / oder Calcineurin-Inhibitoren Therapie, wobei das erfindungsgemäße Pflanzenextrakt in einer pharmazeutischen oder topischen Zusammensetzung dem Patienten verabreicht wird.

Ein weiterer Aspekt der vorliegenden Erfindung betrifft die Verwendung von Cannabinoiden bzw. der erfindungsgemäßen Pflanzenextrakte für die Herstellung eines Arzneimittels zur Behandlung von Neurodermitis (atopische Dermatitis), Allergien, entzündlichen, juckenden Dermatosen, Mückenstichen, Hautatrophien, allergischer Rhinitis, Privinismus, Otitis externa, Asthma bronchiale, Morbus Crohn, Colitis ulcera, Sarkoidose, Konjunktivitis, Mykosen oder entzündlich-rheumatische Erkrankungen oder der Prävention oder Behandlung von phototoxischen Reaktionen. Diese Indikationen können ebenfalls gemeinsam behandelt werden.

Vorzugweise werden die Cannabinoide bei topischen Anwendungen zusammen mit Filaggrin verwendet.

Besonders bevorzugt ist jedoch die Behandlung der Neurodermitis und Mycosen, ggfs. gemeinsam vorliegend, mittels den erfindungsgemäßen Pflanzenextrakten oder Cannabinoiden.

In einer weiteren Ausführungsform betrifft die Erfindung die Behandlung von akuten Schüben der Neurodermitis, die ggfs. einhergehen mit starkem Juckreiz. In diesen Fällen erweist sich eine Dosierung von 0,1 - 5g/l, insbesondere 0,5-1g/l Pflanzenextrakt in einem Lösungsmittel, vorzugsweise Isopropanol als geeignet die akuten Schübe zu behandeln. Ferner kann eine feste Zusammensetzung gewählt werden, die 2-70 %, insbesondere 5 bis 10 % des erfindungsgemäßen Pflanzenextrakts enthält.
Daher eignen sich die erfindungsgemäßen Pflanzenextrakte besonders zur Behandlung von Neurodermitis, da vorteilhaft eine Juckreizstillung erfolgt. In einer weiteren bevorzugten Ausführungsform soll der Pflanzenextrakt zur Nachbehandlung in einer geringeren Dosierung (z.B. 1/3 oder 1/2) angewendet werden.

Die Erfindung soll nun im Folgenden anhand von Beispielen verdeutlicht werden, ohne jedoch auf diese Beispiele beschränkt zu werden.

### 1. Herstellung des Extraktes

1.1. Ausgangsmaterial: Den Ausgangsstoff bilden nach der Ernte bevorzugt getrocknete Blüten und/oderblütennahe Blätter der THC-armen Nutzhanfsorte (z. B. Sorte Futura 75) Diese werden in trockener Luft ("Dachboden") und bei niedrigen Temperaturen (< 35 Grad Celsius) an Leinen nach unten hängend getrocknet.
1.2. Aufbereitung: Bei der Aufbereitung werden die trockenen Blüten und blütennahen Blatter zunächst fein gemahlen. Das fein gemahlene Granulat wird anschließend im Verhältnis von 1:4 bis 1:5 (Volumenverhältnisse) in verschiedene Lösungsmittel eingebracht und verbleibt in diesen für 24h bis 48h in lichtdichten braunen Flaschen zum Extraktionsvorgang bei Zimmertemperatur, jedoch nicht höher.

Der Extrakt wird anschließend abfiltriert und in lichtdichten Flaschen bei etwa 2 Grad Celsius gelagert.

Für die Herstellung des Extraktes eignen sich je nach Verwendungszweck wässrige Auszüge mit Wasser oder alkoholische Extrakte aus Ethanol sowie Isopropanol. Diese sollten bis zur Entwicklung geeigneter Stabilisatoren -ebenso wie die sich aus ihnen herstellbaren und klinisch hochwirksamen Cremes (siehe unten) -gekühlt aufbewahrt werden. Ein im Verhältnis 1:5 verdünnter wässriger Extrakt eignet sich hervorragend für Nasen-, Augen-und Ohrentropfen. Aus dem Isopropylextrakt lassen sich Haartinkturen zur Behandlung von Erkrankungen der Kopfhaut herstellen. Der ethanolische Extrakt ist am besten für die Herstellung von Zubereitungen (z.B. Cremes) für die übrigen Hautbereiche geeignet, wobei es sich auch hier bewährt hat, die Behandlung bei besonders ausgeprägten Symptomen, insbesondere Juckreiz, mit einer ethanolischen oder isopropanolischen Tinktur zu beginnen. Für die Behandlung von Mykosen sind initial, also für die ersten Behandlungstage, bis die oberflächlich sichtbaren Effloreszenzen abgeklungen sind, ebenfalls Tinkturen (unverdünnt) Mittel der Wahl, weil sie zu einem besonders raschen Wirkungseintritt fuhren.

### 2. Klinische Wirksamkeit

### 2.1. Allgemeines

Bei keiner einzigen der im Folgenden beschriebenen klinischen Anwendungen im Rahmen ärztlicher Heilversuche wurden zusätzlich topische oder systemische Arzneimittel, wie z.B. Antihistaminika, Cortisonpräparate oder Calcineurininhibitoren ergänzend eingesetzt.

Alle behandelten Patienten wurden über den ärztlichen Heilversuchscharakter ihrer individuell titrierten topischen Pflanzenextrakttherapie aufgeklärt und auf die konventionell verfügbaren therapeutischen Alternativen hingewiesen, mit denen sie meist bereits mit unbefriedigendem Ergebnis vorbehandelt waren. Alle Patienten bevorzugten die Behandlung mit dem Pflanzenextrakt der vorliegenden Erfindung bzw. einer Creme, in der dieser Pflanzenextrakt eingearbeitet wurde.
Bei keinem der teilweise bis zu 11 Monaten und bei Symptomarmut auch intermittierend behandelten Patienten traten unerwünschte Arzneimittelwirkungen, insbesondere Allergisierungen, auf.
Alle Patienten baten um Auskunft, wie sie auch nach dem ärztlichen Heilversuch in den Genuss der THC-armen Cannabis-Präparate gelangen könnten.

### 2.2. Behandlung von Neurodermitis:

Im Rahmen ärztlicher Heilversuche wurden 21 Patienten unterschiedlichen Alters mit chronischer schwerer Neurodermitis im Durchschnitt 7 bis 10 Tage behandelt. Allen Patienten gemeinsam war:
Chronischer schwerer Verlauf mit unerträglichem Juckreiz, Vorbehandlung mit Cortisonen sowie Calcineurin-Inhibitoren mit unzureichender Wirkung bzw. Nebenwirkungsprovoziertem Behandlungsabbruch oder Verweigerung der Patienten gegenüber einer (weiteren) Cortisontherapie oder Behandlung mit Calcineurin-Inhibitoren.
Bei allen Patienten kam es zu einer deutlichen Linderung bzw. Abklingen des Leitsymptoms Juckreiz. Der Wirkungseintritt begann innerhalb von Minuten nach der ersten topischen Applikation. Die Wirkungsdauer betrug ca. 12 -24h. Die wesentlichsten Effloreszenzen bildeten sich in 3 -7 Tagen zurück. Soweit die Patienten dank der Symptomlinderung bzw. - Befreiung die Fortsetzung der lokalen Behandlung vergaßen, traten die Krankheitssymptome nach Tagen bis Wochen wieder auf, klangen aber unter erneuter Behandlung ebenso rasch wie deutlich ab, wie unter der Ersttherapie. Unter den 21 mit dem Extrakt behandelten Patienten gab es nicht einen einzigen Therapieversager.

### 2.3. Behandlung von Mykosen

Im Fall einer stark übergewichtigen 18-jährigen Patientin kam es zu einer juckenden Mykose im Bereich der linken Submammalfalte, also einem intertriginösen Raum. Die zweimalige Applikation einer pharmazeutischen Zusammensetzung gemäß der Erfindung pro Tag führte bereits nach 2 Tagen zu einer deutlichen und auch anhand der Effloreszenzen optisch nachvollziehbaren Symptombesserung. Die Symptome waren nach einer Woche weiterer Behandlung vollständig abgeklungen. Normalerweise wird unter topischer antimykotischer Therapie eine Dauer von mindestens 14 Tage angesetzt. Ein Zezidiv trat in den folgenden seitdem überblickbaren 5 Sommermonaten nicht mehr auf.

Die konventionelle Therapiedauer von 14 Tagen wurde bei einem 58-jährigen männlichen Patienten mit einer frisch aufgetretenen *Tinea pedis* mit starken Verhornungen im Fersenbereich angewandt, um auch in tiefer gelegenen Hautarealen antimykotisch wirksame Konzentrationen zu erreichen. Neben mechanischer Abtragung der verhornten Hautareale wurde bei diesem Patienten in den ersten drei Behandlungstagen der unverdünnte (im Verhältnis 1:4 angesetzter) frische Pflanzenextrakt der vorliegenden Erfindung 2x täglich aufgetragen. Nach dem Eintrocknen wurde diese Behandlung durch die Applikation einer Creme, die diesen Extrakt enthält, ergänzt. Vom 3. bis zum 14. Behandlungstag wurde die Creme nur noch 1 -2 mal täglich appliziert und nach einer Einwirkungsdauer von ca. 1 Stunde abgewischt, um Wäscheverschmutzungen auf Basis des in den Versuchschargen noch nicht eliminierten Chlorophylls zu vermeiden. Nach dieser Behandlung kam es zu einer vollständigen Beseitigung der Mykose.

Eine 57-jährige Patientin, die schon seit ihrer Jugend genetisch bedingt unter Neurodermitis leidet, berichtet seit Jahren über immer wieder rezidivierende Hautmykosen im Bereich der Neurodermitis-bedingten Schädigungen des Integuments insbesondere im vorher immer wieder befallenen intertriginösen pedalen Interdigitalbereich. Seitdem die Patientin mit den topischen Zubereitungen des Pflanzenextrakts der vorliegenden Erfindung behandelt wird, intermediär inzwischen 11 Monate, trat kein einziges Rezidiv einer Mykose auf.

### 2.4. Behandlung von Urticaria

Bei drei Patienten traten unter Gartenarbeit (Unkrautjäten in Staudenbeeten) Urticaria an beiden Unterarmen mit ausgeprägten Exanthemen und heftigstem Juckreiz auf. Diese Symptome konnten mit sofort aufgetragener Creme aus *unguentum alcoholes lanae* (DAB 998), in der der alkoholische Pflanzenextrakt der vorliegenden Erfindung bis zur Sättigung eingebracht war, innerhalb von 10 -30 Minuten (Juckreiz) und 30 -60 Minuten (Exanthem) nach einmaliger Applikation vollständig und anhaltend zum Abklingen gebracht werden.

### 2.5. Behandlung von Mückenstichen

Mückenstiche mit heftigen ca. 5-Markstück-grossen urtikariellen Höfen konnten in 4 Fällen bei Patienten unterschiedlichen Alters mit einmaliger Applikation einer Creme, die den Pflanzenextrakt der vorliegenden Erfindung enthält, bis zum vollständigen und nachhaltigen Abklingen gebracht werden.

Bei einem weiteren männlichen 55-jährigen Patienten kam es nach einem etwa 3 cm links paraumbilikalem Insektenstich zu einer extrem ausgeprägten Urticaria im Bereich des gesamten linken Mittel-und Unterbauches. Der Befund war so schwerwiegend, dass man in diesem Fall, ohne einer klinischen Vorerfahrung mit dem Pflanzenextrakt der vorliegenden Erfindung, sehr wahrscheinlich lokal und systemisch mit Dexamethason (ein besonders wirkungsstarkes Cortison) behandelt hätte. Nachdem bei dem Patienten jedoch keine Anzeichen eines beginnenden anaphylaktoiden Schocks erkennbar waren, wurde, unter ärztlicher Aufsicht, zunächst ein Behandlungsversuch mit dem Pflanzenextrakt der vorliegenden Erfindung bzw. einer Creme, die diesen Pflanzenextrakt enthält, durchgeführt. Bereits nach ca. 20 -30 Minuten, war der Juckreiz weitgehend abgeklungen, die Quaddel an der Einstichstelle deutlich zurückgegangen, die Schwellung zurückgebildet und das Exanthem ebenfalls abgeblasst. Zwar kehrten bei diesem Patienten über die nächsten 5 Tage Juckreizepisoden zurück und auch das Exanthem war noch nicht vollständig verschwunden, jedoch reagierten diese Restsymptome regelmäßig auf die erneute Applikation der Creme.

### 3. Möglicher Wirkungsmechanismus

Der rasche Wirkungseintritt sowie die lange Wirkdauer des Pflanzenextraktes der vorliegenden Erfindung sprechen für zwei verschiedene Angriffspunkte:
1. Der rasche, innerhalb von Minuten stattfindende, Wirkungseintritt lässt vermuten, dass der Pflanzenextrakt unter anderem mit den Juckreiz-Rezeptoren des sensiblen Nervensystems der Haut reagiert. Hierfür spricht, dass zu diesem Zeitpunkt die Mediatoren der Mastzellen (z.B. Histamine und Leukotriene) bereits ausgeschüttet sind und in dem Hautgewebe die quälenden Symptome verursachen. Ein Angriffspunkt an den entsprechenden Rezeptoren darf schon deshalb angenommenen werden, da die symptomatische Erleichterung durch Kratzen über den Hautdefekt ebenfalls diese Rezeptoren zerstört. Dadurch können keine Juckreizerzeugende Aktionspotentiale mehr abgefeuert werden. Daraus folgt, dass auch der kurzfristig wirksame lokale Effekt des Pflanzenextrakts über diese Rezeptoren, wenn auch nicht durch deren Zerstörung, so doch z. B. über einen möglichen kompetitiven Effekt zu erklären ist.
2. Die lange Wirksamkeitsdauer ist dagegen über einen gänzlich anderen Wirkungsmechanismus erklärbar. Es handelt sich hierbei wahrscheinlich um eine Pflanzenextrakt-bedingte Sekretionshemmung der Histamine und Leukotriene aus den Mastzellen. Hierfür spricht, dass an den Mastzellen des Menschen bereits zwei Typen von Endocannabinoidrezeptoren nachgewiesen wurden. Damit ist auch nachgewiesen, dass die Schaltzentrale aller zellulär vermittelten immunologischen Reaktionen, die Mastzelle, durch Cannabinoide maßgeblich beeinflusst werden kann. Aus dieser Erkenntnis folgt, dass auch die anderen in dieser Erfindung angesprochenen Erkrankungen, die ebenfalls über Mastzellreaktionen vermittelt werden, günstig beeinflusst werden.
3. Aus diesen Überlegungen folgt weiter, dass sämtliche, über Mastzellen vermittelten Immunreaktionen durch Cannabinoide günstig beeinflusst werden.

## Patentansprüche

1. Mittel enthaltend ein Pflanzenextrakt aufweisend einen THC - Gehalt von weniger als 0,5 Gew. % aus den Blüten und/oder Blättern und/oder Stengeln und/oder Wurzeln und/oder Samen einer Tetrahydrocannabinol-(THC-)armen Sorte von *Cannabis sativa subsp. sativa,*
zur Verwendung in der Behandlung von Juckreiz.

2. Mittel enthaltend ein Pflanzenextrakt aufweisend einen THC - Gehalt von weniger als 0,5 Gew. % aus den Blüten und/oder Blättern und/oder Stengeln und/oder Wurzeln und/oder Samen einer Tetrahydrocannabinol-(THC-)armen Sorte von *Cannabis sativa subsp. sativa,*
zur Verwendung in der Behandlung von Juckreiz nach Anspruch 1, wobei eine Erkrankung ausgewählt ist aus der Gruppe Neurodermitis, Kontaktekzem, Allergien, der Prävention oder Behandlung von phototoxischen Reaktionen, Rosazea, perioraler Dermatitis, Akne, Akne conglobata, Mückenstichen, Hautatrophien, Cortison-bedingte Hautveränderungen, Mykosen, Urtricaria, allergischer Rhinitis, Prvinismus, Konjunktivitis, Otitis externa, Colitis ulcera, Sarkoidose.

3. Mittel enthaltend ein Pflanzenextrakt aufweisend einen THC - Gehalt von weniger als 0,5 Gew. % aus den Blüten und/oder Blättern und/oder Stengeln und/oder Wurzeln und/oder Samen einer Tetrahydrocannabinol-(THC-)armen Sorte von *Cannabis sativa subsp. sativa,*
zur Verwendung in der Behandlung von Juckreiz nach Anspruch 1, wobei eine Erkrankung ausgewählt ist aus der Gruppe Neurodermitis, Mykosen, Mückenstichen.

4. Mittel zur Verwendung in der Behandlung von Juckreiz nach einem der vorhergehenden Ansprüche, wobei als Extraktionsmedium Lösungsmittel und/oder Gemische von Lösungsmitteln verwendet werden, wobei die Lösungsmittel ausgewählt sind aus der Gruppe bestehend aus Wasser, Kochsalzlösung, Alkohol, Aceton, Estern und Ethern.

5. Mittel zur Verwendung in der Behandlung von Juckreiz nach einem der vorhergehenden Ansprüche, wobei als Extraktionsmedium 0,9 % Kochsalzlösung, 90% Äthanol oder 70% Isopropanol verwendet wird.

6. Mittel zur Verwendung in der Behandlung von Juckreiz nach einem der vorhergehenden Ansprüche, wobei die THC-arme Sorte *Cannabis sativa subsp. sativa* die Sorte Futura 75 ist.

7. Mittel zur Verwendung in der Behandlung von Juckreiz nach einem der vorhergehenden Ansprüche, wobei der THC - Gehalt weniger als 0,2 % oder 0,1 oder 0 % (Gew. %) THC - Gehalt aufweist.

8. Pharmazeutische Zusammensetzung umfassend ein Mittel zur Verwendung in der Behandlung von Juckreiz nach einem der vorhergehenden Ansprüche.

9. Mittel zur Verwendung in der Behandlung von Juckreiz nach einem der Ansprüche 1 bis 7 oder einer pharmazeutischen Zusammensetzung nach Anspruch 8 zur Verwendung in der Behandlung von Therapieversagern und/oder Verweigerern einer Therapie mit Cortison-Präparaten und/oder Calcineurin-Inhibitoren sowie von Nebenwirkungen von Cortison-Präparaten und/oder Calcineurin-Inhibitoren oder zur Ersatztherapie zur Behandlung mit Cortison-Präparaten und/oder Calcineurin-Inhibitoren.

## Claims

1. Preparation containing a plant extract having a THC content of less than 0.5 weight % made from the flowers and/or leaves and/or stems and/or roots and/or seeds of a low-tetrahydrocannabinol (THC) variety of *Cannabis sativa subsp. sativa,*
for use in the treatment of itching.

2. Preparation containing a plant extract having a THC content of less than 0.5 weight % made from the flowers and/or leaves and/or stems and/or roots and/or seeds of a low-tetrahydrocannabinol (THC) variety of *Cannabis sativa subsp. sativa,*
for use in the treatment of itching according to claim 1, a disease being selected from the group comprising neurodermatitis, contact dermatitis, allergies, the prevention or treatment of phototoxic reactions, rosacea, perioral dermatitis, acne, acne conglobata, mosquito bites, atrophy of the skin, cortisone-induced skin changes, mycoses, urticaria, allergic rhinitis, rhinitis medicamentosa, conjunctivitis, otitis externa, ulcerative colitis, sarcoidosis.

3. Preparation containing a plant extract having a THC content of less than 0.5 weight % made from the flowers and/or leaves and/or stems and/or roots and/or seeds of a low-tetrahydrocannabinol (THC) variety of *Cannabis sativa subsp. sativa,*
for use in the treatment of itching according to claim 1, a disease being selected from the group neurodermatitis, mycoses, and mosquito bites.

4. Preparation for use in the treatment of itching according to one of the preceding claims, the extractant used being solvents and/or solvent mixtures, the solvents being selected from the group consisting of water, saline solution, alcohol, acetone, esters, and ethers.

5. Preparation for use in the treatment of itching according to one of the preceding claims, the extractant used being 0.9% saline solution, 90% ethanol, or 70% isopropanol.

6. Preparation for use in the treatment of itching according to one of the preceding claims, the low-THC variety of *Cannabis sativa subsp. sativa* being the variety Futura 75.

7. Preparation for use in the treatment of itching according to one of the preceding claims, wherein the THC content has a THC content less than 0.2% or 0.1 or 0% (weight %).

8. A pharmaceutical composition comprising a preparation for use in the treatment of itching according to any one of the preceding claims.

9. A preparation for use in the treatment of itching according to any one of the claims 1 through 7 or a pharmaceutical composition according to claim 8 for use in the treatment of patients in whom therapy with cortisone preparations and/or calcineurin inhibitors fails or who refuse such therapy, and for use in the treatment of side effects of cortisone preparations and/or calcineurin inhibitors or for replacement therapy for treatment with cortisone preparations and/or calcineurin inhibitors.

## Revendications

1. Produit contenant un extrait de plantes présentant une teneur en tétra hydro cannabinol, THC, inférieure à 0,5 % en poids, à base de fleurs, et/ou de feuilles, et/ou de tiges, et/ou de racines, et/ou de graines d'une espèce pauvre en tétra hydro cannabinol (THC) de *Cannabis sativa subsp. sativa,*
pour l'utilisation dans le traitement de démangeaisons.

2. Produit contenant un extrait de plantes présentant une teneur en THC inférieure à 0,5 % en poids, provenant des fleurs, et/ou des feuilles, et/ou des tiges, et/ou des racines, et/ou des graines d'une espèce pauvre en tétra hydro cannabinol (THC) de *Cannabis sativa subsp. sativa,*
pour l'utilisation dans le traitement de démangeaisons selon la revendication 1, où une maladie est choisie dans le groupe de la neurodermite, des eczémas de contact, des allergies, dans la prévention ou le traitement des réactions phototoxiques, de la rosacée, de la dermatite péri-orale, de l'acné, de l'acné conglobata, des piqûres de moustiques, d'atrophies cutanées, de modifications cutanées liées à la cortisone, de mycoses, d'urticaire, de la rhinite allergique, de privinisme, d'une conjonctivite, d'une otite externe, de la colite ulcéreuse, de la sarcoïdose.

3. Produit contenant un extrait de plantes présentant une teneur en THC inférieure à 0,5 % en poids, provenant des fleurs, et/ou des feuilles, et/ou des tiges, et/ou des racines, et/ou de graines d'une espèce pauvre en tétra hydro cannabinol THC de *Cannabis sativa subsp. sativa,*
pour l'utilisation dans le traitement de démangeaisons selon la revendication 1, où une maladie est sélectionnée dans le groupe de la neurodermite, des mycoses, des piqûres de moustiques.

4. Produit destiné à l'utilisation dans le traitement de démangeaisons selon l'une des revendications précédentes, dans lequel, en tant que milieu d'extraction, des solvants et/ou des mélanges de solvants sont utilisés, où les solvants sont choisis dans le groupe constitué de l'eau, d'une solution de sel de cuisine, d'un alcool, de l'acétone, d'esters et d'éthers.

5. Produit pour l'utilisation dans le traitement de démangeaisons selon l'une des revendications précédentes, dans lequel, en tant que milieu d'extraction, on emploie une solution de sel de cuisine à 0,9 %, de l'éthanol à 90 % ou de l'isopropanol à 70 %.

6. Produit destiné à l'utilisation dans le traitement de démangeaisons selon l'une des revendications précédentes, où l'espèce pauvre en THC de *Cannabis sativa subsp. sativa* est l'espèce Futura 75.

7. Produit destiné à l'utilisation dans le traitement de démangeaisons selon l'une des revendications précédentes, dans lequel la teneur en THC présente une teneur en THC inférieure à 0,2 %, ou à 0,1 ou à 0 % (% en poids).

8. Composition pharmaceutique comprenant un produit destiné à l'utilisation dans le traitement de démangeaisons selon l'une des revendications précédentes.

9. Produit destiné à l'utilisation dans le traitement de démangeaisons selon l'une des revendications 1 à 7 ou composition pharmaceutique selon la revendication 8 pour l'utilisation dans le traitement d'échecs thérapeutiques et/ou de personnes refusant une thérapie avec des préparations à la cortisone et/ou des inhibiteurs de la calcineurine, ainsi que dans des effets secondaires des préparations à la cortisone et/ou des inhibiteurs de la calcineurine, ou pour la thérapie de substitution pour le traitement avec des préparations à la cortisone et/ou des inhibiteurs de calcineurine.
